# EUROPEAN PATENT APPLICATION

(11) **EP 1 500 708 A1**
(43) Date of publication of application: **26.01.2005**
(21) Application number: 03016547.6
(22) Date of filing: 24.07.2003
(51) Int. Cl.: C12Q 1/68

(54) **Assay for detection of emetic toxin producing Bacillus cereus**

(71) Applicant: Scherer, Siegfried, Prof. Dr., 85354 Freising (DE)
(72) Inventor: Ehling-Schulz, Monika, Dr., 81539 München (DE); Fricker, Martina, 93055 Regensburg (DE); Scherer, Siegried, Prof. Dr., 85354 Freising (DE)

(57) **Abstract**

The present invention is an assay for detection and identification of emetic toxin producing *B. cereus* strains. The assay is based on polymerase chain reaction (PCR) techniques and also relates to diagnostic kits for detection of emetic *B. cereus* in samples.

## Description

### Background of the invention

*Bacillus cereus* is a gram positive spore forming food pathogen that causes two types of food poisoning syndromes: emesis and diarrhea. It is an increasing problem especially in heat treated foods, such as convenience foods and foods used in catering because of its resistance to pasteurization and anti-microbial agents. For instance, between 1980 and 1997, 2715 cases of *B*. *cereus* food poisoning in England and Wales were reported to the Public Health Laboratory Services (PHLS) and it was the most common pathogen found in 3% of the samples taken from hot meals served in aircraft from 1991 to 1994 (Hatakka 1998). In general, the incidence of *B. cereus* food poisoning is underestimated since *B. cereus* is not a reportable disease and reporting procedures are varying between countries. There is a tendency that in northern countries, much more *B*. *cereus* food poisoning cases are reported. In Norway *B*. *cereus* was the most common microbe isolated from food borne illness in 1990 (Aas *et al*., 1990). While in Norway the diarrheal type of food poisoning was predominent, the emetic type was prevalent in the United Kingdom, in Japan (Kramer and Gilbert 1989) and in the United States (Hall *et al*., 2001). The true incidence of *Bacillus cereus* food poisoning is unknown for a number of reasons, including poor responses from victims during interviews with health officials; misdiagnosis of the illness, which may be symptomatically similar to other types of food poisoning (such as vomiting caused by *Staphylococcus aureus* toxin), inadequate collection of samples for laboratory analyses and improper laboratory examination. Some countries, e.g. Belgium (Anonymous 2001), report *B*. *cereus* and *S. aureus* infections together because discrimination of these two food poisoning organism is rather difficult and laborious. Since no molecular tools are available that allow a rapid identification of emetic *B*. *cereus,* time consuming cultivation would be necessary to discriminate these two organism, which are often involved in food poisoning caused by consumption of pasta. Like the enterotoxins of *S*. *aureus* the emetic toxin of *B. cereus* is heat stable and toxic at low doses (Hoomstra *et al*., 2003).

The emetic syndrome is mainly characterized by vomiting a few hours after ingestion of the contaminated food. In the diarrheal syndrome, symptoms appear 8 to 16 hours after ingestion, and include abdominal pain and diarrhea. While the emetic type of food poisoning caused by *B*. *cereus* resembles staphyloenterotoxicosis, the diarrheal syndrome of *B*. *cereus* resembles the symptoms of a *Clostridium perfringens* infection. In general, both types of food borne illness caused by *B*. *cereus* are relatively mild and usually do last not more than 24 hours. Nevertheless, more severe forms have occasionally been reported, including one death after the ingestion of food contaminated with high amounts of emetic toxin and three deaths caused by a necrotic enterotoxin (Lund *et al*., 2000; Mahler *et al*., 1997).

Different enterotoxins are the causative agents for the diarrhoeal syndrome. These are comparatively well characterized at the molecular level (for review see Granum, 2001) and molecular diagnostic assays at an immunological and at a PCR level are available (e.g. Granum and Lund, 1997; Hansen and Hendriksen, 2001). Far less is known about the emesis causing cereulide. Cereulide is a small, heat stable dodecadepsipeptide which, for instance, was shown to be involved in fulminate liver failure in a human case (Mahler *et al.,* 1997). It has been shown to cause cellular damaging effects in animal models and to be toxic to mitochondria by acting as a potassium ionophore (Agata *et al*., 1995; Mikkola *et al.,* 1999; Shinagava *et al*., 1995; Shinagava *et al*., 1996). Recently, it has been reported that cereulide inhibits human natural killer cells and might therefore have an immunomodulating effect (Paananen *et al*., 2002). Enterotoxin production is commonly found among the different members of the *B*. *cereus* group whereas emetic toxin production is restricted to a certain group *of B. cereus* strains. There is some evidence for a clonal population structure of cereulide producing emetic isolates while enterotoxin producing *B*. *cereus* isolates are quite polymorphic (Ehling-Schulz *et al*., *2003).*

The emetic toxin is associated with different vegetable foods, especially rice and pasta but it has been isolated from baby food, too. There are three possibilities to detect the emetic toxin: via cytotoxicity, HPLC-MS and using a sperm-based bioassay (Andersson *et al*., 1998; Finlay *et al*., 1999; Häggblom *et al.* 2002). These assays are rather difficult to be performed at a routine basis and need one day to one week with precultivation and laborious sample preparations. With exception of HPLC-MS, these assays do not specifically detect cereulide; e.g. the bioassay is also sensitive to other mitochondrial toxins like gramicidin (Andersson *et al*., 1998).

Besides its food poisoning potential *B*. *cereus* has been shown to be responsible for wound and eye infections, as well as systemic infections and periodontitis (Beecher *et al*., 2000; Drobniewski, 1993; Helgason *et al*., 2000). It is increasingly being identified as the cause of serious or life-threatening infections in neutropenic and immunosuppressed patients and premature neonates (Thuler *et al*., 1998; Musa *et al*., 1999; Hilliard *et al*., 2003). There are only few reports of fatal *B. cereus* bacteremia in adults with acute leukemia, but all patients in theses reports demonstrated gastrointestinal symptoms immediately preceding death (Akiyama *et al*., 1997; Ginsburg *et al*., 2003). Recently, it has been reported that systematic complications of *B*. *cereus* infection in premature neonates might be at least partly related to enterotoxins (Girisch *et al*., 2003). However, in general the role of the diverse toxins and virulence factors of *B*. *cereus* in systemic infections is poorly studied. The development of molecular tools will be necessary to allow a rapid characterization of virulence mechanisms of clinical *B. cereus* isolates.

Since *B*. *cereus* is on ubiquitous spore former that can not totally be avoided, it is necessary to develop methods for a rapid discrimination of hazard strains from non toxic strains. The utility of PCR based methods is evident by the recent guidelines issued by NCCLS (National Committee for Clinical Laboratory Standards, USA) in 1999 encouraging the use of molecular methods in clinical laboratories performing bacterial identification assays (Anonymous 1999). Such an assay would also be advantageous for quality control in food industry and could improve food safety substantially. For enterotoxic *B. cereus* strains molecular diagnostic assays at PCR level have been described (e.g., Prüß *et al*., 1999, Hansen and Hendriksen 2001, Guinebretiere *at al*., 2002) and immunological assays are commercial available (e.g., for detection of the L2 component of Hbl (HblC) the BCET-RPLA *B*. *cereus* Enterotoxin Test Kit (Oxoid, Basingstoke, UK) and for detection of NheA the Tecra BDE kit (Tecra Diagnostics, Frenchs Forest, Australia)) while such tools for emetic strains are still missing.

The present invention fills that gap by providing a molecular assay to rapidly detect emetic toxin producing *B. cereus* strains.

### Brief summary of the invention

Many strains of *Bacillus cereus* can cause gastrointestinal diseases. The emetic type of the disease is attributed to a heat stable depsipeptide called cereulide and resembles *Staphylococcus aureus* food poisoning. Since the molecular basis for cereulide synthesis in *vivo* has not been elucidated yet, no molecular tools for a rapid detection/identification of hazard emetic *B*. *cereus* strains were available. PCR amplification using specific primers would enable a fast detection of emetic *B*. *cereus* in food and clinical samples.

Based on a PCR screening technique, we have identified a PCR fragment of unknown function which, unexpectedly, turned out to be specific for emetic strains of *B*. *cereus.* Subsequently, the entire sequence of this amplicon was determined and on this basis, a PCR assay was developed. 179 *Bacillus cereus* isolates obtained from different food poisoning outbreaks and diverse food sources from various geographical locations and 29 strains from other species belonging to the *B*. *cereus* group were tested using this assay. The assay turned out to be specific for emetic toxin producing *B*. *cereus* strains. None of the non emetic B. *cereus* strains showed any false positive response. In addition, 49 non *B. cereus* group strains, with special emphasis of food pathogens, were tested to prove the specificity of the assay (see table1). No PCR amplificons from non *B. cereus* species were observed.

The invention is a molecular assay that allows a rapid detection of emetic *B*. *cereus* strains within a day and offers the basis for the development of molecular assay for a fast discrimination of, e.g., enterotoxigenic *Staphylococcus aureus* and emetic *B. cereus.*

### Brief description of the drawings

Figure 1. Gel electrophoresis of the PCR amplification products from purified template DNA of emetic and non emetic *Bacillus cereus* isolates using degenerated primers.
Figure 2. Gel electrophoresis of the PCR products amplified with the primer set 800F2 and 800R2 from purified DNA of emetic and non emetic *Bacilli* and other bacterial species involved in food poisoning (example1). 16SrDNA of samples was amplified in parallel as a control.
Figure 3. Gel electrophoresis of the PCR amplification of template DNA from microbial mixtures of example 2.

### Detailed description of the invention

The chemical structure of cereulide, the emesis causing toxin of *B*. *cereus,* has been solved several years ago (Agata *et al*., 1994), but the mechanism of its synthesis remained unknown. Genetic loci responsible for enterotoxin production have been sequenced, characterized and used to set up molecular detection systems (for review see Granum 2001), while the genes responsible for the emetic toxin production are unknown and no molecular detection systems are available. The development of a nucleic acid amplification system that specifically detects emetic *Bacillus cereus* without cross reacting with other organism is very challenging.

Cereulide is a small cyclic dodecadepsipeptide that shows vacuole formation activity in HEp-2 cells (Isobe et al., 1995). Based on its structure and function it might be, like many other natural peptides and depsipeptides (Kleinkauf and Von Döhren 1996; Stachelhaus and Marahiel 1995), synthesized enzymatically by a nonribosomal peptide synthase. These peptide synthases share considerable homologies in active center structure over a wide taxonomic range (Marahiel *et al*., 1997, Von Döhren *et al*., 1997) which makes it possible to design degenerated PCR primers. These primers can be used in screening assays for amplification of parts from these genes and to detect their sequences.

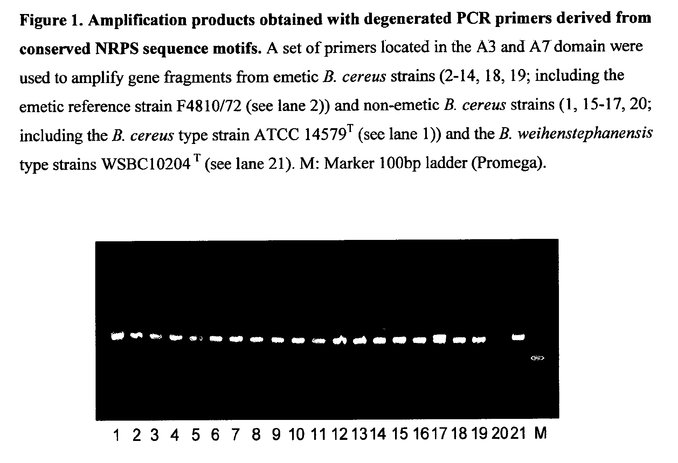

In search of the molecular basis of emetic toxin production degenerated primers targeting conserved motifs of peptide synthases were used to amplify fragments from potential non ribosomal peptide synthases from different *B. cereus* isolates. With a primer combination targeting the A3 and A7 domain two distinct bands, 700bp and 800bp, for the emetic *B*. *cereus* strains but only one band (700bp) for the non emetic *B. cereus* strains were obtained (see Figure 1). The 800bp fragment that was amplified from the cereulide producing *B*. *cereus* strain F4810/72 was cloned into TOPO TA (Invitrogen) and sequenced. The resulting sequence was searched against NCBI's non-redundant database using BLAST (Altschul *et al*., 1990), and it was used to design primers and to set up a PCR assay for detection of emetic *B. cereus* isolates.

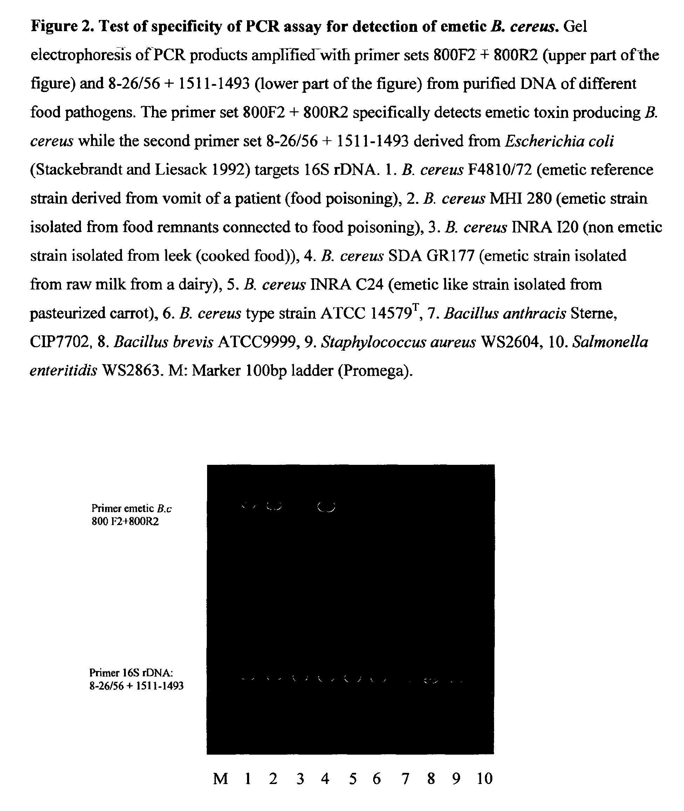

Database search using BLAST revealed some homologies of the 800bp fragment to AMP binding genes and Acetyl CoA synthase, but no significant homologies to known peptide synthases. The sequence of this gene fragment was used to develop a PCR assay to specifically detect emetic *B*. *cereus* strains (Figure 2).

The primers that have been provided are (see sequence list seq no. 1 and 2): 800F2: 5′-GACAAGAGAAATTTCTACGAGCAAGTACAAT-3′ and

800R2: 5′-GCAGCCTTCCAATTACTCCTTCTGCCACAGT-3′

The system, using this primer combination, was tested on 179 *B*. *cereus* isolates from clinical cases, foods and environments and cross reactivity was tested for closely related members of the *B*. *cereus* group as well as for other Bacilli and known food pathogens. In total, 208 *B. cereus* group strains and 49 isolates from other species were tested. Figure 2 shows results of the PCR amplification of target DNA with the above mentioned primer combination of the invention and results of control PCR using standard primer targeting 16 S rDNA (Stackebrandt and Liesack 1992). No cross reaction with the above mentioned primers (800F2 and 800R2) were observed while all emetic toxin producing strains were detected (see Table 2). Interestingly, no cross reaction were observed to emetic like strains that are closely related to emetic strains. The term 'emetic like strains' refers to *B*. *cereus* strains that share biochemical properties with emetic *B*. *cereus* strains and that are clustering together with emetic strains by genetic and phenetic methods (Ehling-Schulz *et al*., 2003), but do not produce the emetic toxin. Since these strains can not be discriminated from emetic strains by genetic methods like RAPD or by phenetic methods like FTIR, other methods are needed for a rapid and reliable discrimination. One of the major challenges of the invented PCR assay is, that it exclusively detects emetic toxin producing strains, but does not cross-react with these closely related non emetic *B*. *cereus* isolate nor does it cross-react with any other bacteria tested.

The primer pair described in the presented invention amplifies a DNA product of 635 bp. However other primer pairs located in the target sequence (see sequence list seq no. 3) might also be suitable for use in the presented invention, e.g. for the development of a multiplex PCR system etc., therefore they are within the scope of the presented invention. Direct sequencing of DNA amplification products of 10 different emetic strains from diverse geographical locations, using the primers of the presented invention, revealed identical sequences of this gene of unknown function for all strains (Ehling-Schulz, unpublished). Southern blot hybridization and database search of sequenced genomes showed that the gene fragment described in the present invention is specifically found in emetic *B. cereus* isolates whereas it is not detected in other bacteria (data not shown).

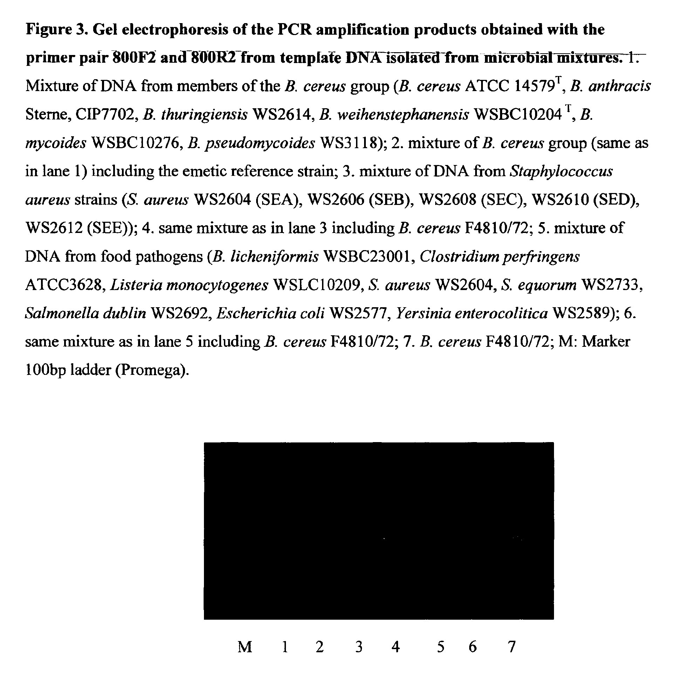

In further testing the specificity of the primer pair, a chromosomal DNA mixture of members of the *B. cereus* group and a DNA mixture of known food pathogens was amplified in the presence and absence of chromosomal DNA from the emetic reference strains F4810/72. The PCR amplicons were run on an agarose gel (see Figure 3). The result showed a high specificity of the primers, amplicons were only observed in the presence of the emetic strain. Therefore the primer pair described in the present invention may be used for detection of emetic *B*. *cereus* isolates in pure or mixed culture; however the usage of these primers is not limited to pure or mixed cultures in this invention.
The present invention also includes a method for the detection of emetic *B*. *cereus* in various samples. The method comprises the following steps: collection of the sample, preparing the sample for nucleic acid amplification, adding reagents for amplification to the prepared sample, including a primer pair of the invention, amplification and detection of emetic *B*. *cereus* in the sample. For the isolation of nucleic acids from any possible sources several techniques are described in the literature (Inglis *et al*., 2003; Lantz *et al*,,.. 2000; Maniatis *et al*., 1998; Ramesh *et al*., 2002; Tsai *et al*., 1991) and those skilled in the art may also design alternative methods for nucleic acid isolation.

The principles and conditions for the performance of nucleic acid amplification and detection steps by using polymerase chain reaction (PCR) of the present invention are well known and provided in numerous references including e.g., U.S. patent Nos 4,683,195 (Mullis *et al*., 1987) 4,902,624 (Columbus *et al.,* 1990); McPherson *et al.,* 1995; Innis *et al*., 1990. Therefore a worker skilled in the art should have no difficulty in practicing the present invention by amplification of the target nucleic acid with the primers (oligonucleotides) provided by the invention to detect emetic toxin producing *B. cereus.*

The target nucleic acids prepared for the amplification is set in contact with the PCR mixture containing at least one oligonucleotide primer pair provided by the invention, DNA polymerase enzyme, DNA polymerase cofactor and on or several deoxynucleoside-5'-triphosphates (dNTPs) under appropriate conditions for the amplification of any nucleic acids from emetic *B. cereus* presented in the sample. Primers used in the present invention might by labeled. Using known methods in the art, the labeling of the primers and probes can be done with specific ligands; e.g., biotin, streptavidin, hapten etc., and enzyme; e.g., glucose oxidase, peroxidase, uricase, alkaline phosphatase; radioisotopes, electro-condense reagents, chromogens, phosphorescent moieties or ferretin. Such labels are within the scope of the invention. The PCR reagents are provided individually or in various combinations, and are used in suitable concentrations and pH to enable amplification of the target nucleic acid.

PCR is, to date, the best method of amplifying regions of DNA and is used to amplify small quantities of target DNA and it is preferably conducted in a continuous automated system. Several instruments have been developed for this purpose and are available for those whom are skilled in the art, such as the chemical test pack described in U.S. patent No. 5,229,297 and processed in the instrument described in U.S. patent No.5,089,233. The assay may also be scaled down to a micro level in order to allow rapid analysis of many samples. Assays can be automated and carried out in microtiter plates.

Following PCR amplification, the presence or absence of the amplified target nucleic acids can be confirmed using known detection methods. In the present invention PCR amplicons were separated by agarose gel electrophoresis, stained with ethidium bromide and visualized by illumination with ultraviolet light. In addition, the amplified target can be detected by probes that are specific for the gene region, e.g., by southern blot, dot blot techniques, or non-isotopic oligonucleotide capture detection with labeled probes.

The invention also includes a kit for use in detecting the presence of emetic toxin producing *B*. *cereus* nucleic acids in a sample by detecting the target nucleic acid sequence.. A kit preferable contains, beside a set of primers of the invention (which in combination amplify the gene of unknown function in emetic *B*. *cereus*) a nucleic acid polymerase and additional reagents needed for the amplification. A kit according to the present invention can be used, e.g., for the detection of emetic toxin producing *B*. *cereus* in foods, clinical or environmental samples.

The PCR based detection system of the present invention provides a basis for the development of multiplex PCR systems to detect either the full set of toxin genes involved in gastrointestinal diseases caused by *B*. *cereus* or on the other hand a multiplex PCR system can be developed for a rapid discrimination of *B*. *cereus* and *Staphylococcus aureus* or other food pathogens. This application is within the scope of the present invention.

**Table 1**

| Bacterial species used to test the specificity of PCR Assay | |
|---|---|
| Bacterial species (no. of species) | No. of strains tested |
| *Bacillus cereus* group (6) | |
| *Bacillus cereus* | 179 |
| *Bacillus anthracis* | 7 |
| *Bacillus thuringiensis* | 6 |
| *Bacillus mycoides* | 6 |
| *Bacillus pseudomycoides* | 3 |
| *Bacillus weihenstephanensis* | 7 |

| Other *Bacillus* sp. (4) | |
|---|---|
| *Bacillus brevis* | 3 |
| *Bacillus subtilis* | 1 |
| *Bacillus licheniformis* | 3 |
| *Bacillus amyloliquefaciens* | 1 |

| Other non *Bacillus* species (9) | |
|---|---|
| *Staphylococcus aureus* | 10 |
| *Staphylococcus equorum* | 1 |
| *Clostridium perfringens* | 3 |
| *Listeria monocytogenes* | 6 |
| *Campylobacter sp.* | 3 |
| *E. coli* (incl. serovar 0157) | 4 |
| *Salmonella* sp. | 6 |
| *Yersinia enterocolitica* | 8 |

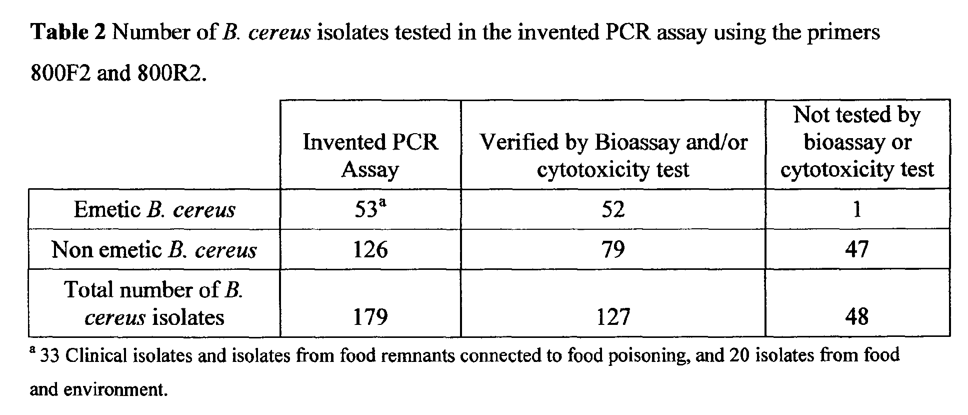

### Examples

### 1.Example: The detection of emetic Bacillus cereus from purified genomic DNA of single colonies or liquid cultures.

### Isolation of microbial DNA.

Bacteria were grown on agar plates or in liquid cultures. DNA from gram positive isolates was prepared using the AquaPure Genomic DNA Isolation-Kit (Biorad, Germany). DNA from gram negative isolates was prepared by suspending cells from one colony in sterile water, boiled for 3 minutes and then placed on ice. After centrifugation at 16.100 rpm for 30 seconds the supernatant was used as template for PCR or stored at - 20 °C. An aliquot of each DNA preparation was analyzed by agarose electrophoresis to assess the DNA content.

### PCR amplification.

PCR mixture (50 µl) contained 0.8 mM dNTPs mix (ABgene, Epsom, U.K), 1.5 mM MgCl₂, 50 pM of each primer, 1.25 U of ThermoStart *Taq* DNA polymerase (ABgene, Epsom, U.K.), 5 µl 10x polymerase buffer (ABgene, Epsom, U.K) and 1 µl template DNA. Thermal cycling was carried out in a Peqlab thermocycler, Cyclone Gradient (Biotechnologie GmbH, Germany). The PCR protocol started with a denaturation step for 15 min at 95°C, followed by 30 cycles of 1 min 95°C, 1.5 min 60°C, 50 s at 72°C each and ended with a final elongation step at 72°C for 5 min. All strains used for the evaluation of the assay were checked with the invented primer set 800F2 and 800R2 and in parallel selected strains were amplified with 16S primers as a positive control. For amplification of 16S rDNA the general primers derived from *Escherichia coli,* 8-26/56: 5'-AGAGTTTGATCCTGGCTCA-3' and 1511-1493: 5'-CGGCTACCTTGTTACGAC-3' (Stackebrandt and Liesack 1992) were used.

### Detection of amplicons.

PCR products were separated on a 1% agarose gel, stained with ethidium bromide and visualized with Image Master VDS (Pharmacia Biotech).

### Evaluation of the PCR assay.

To test the specificity of the PCR assay purified genomic DNA from 179 *B*. *cereus* isolates, 29 isolates from other member of the *B. cereus* group closely related to *B*. *cereus* and type strains of other *Bacillus* species were included in this study. In addition, 43 isolates from other bacterial species were analyzed (see Table 1). 53 out of 53 cereulide producing *B*. *cereus* isolates were detected by the assay while no non cereulide (emetic toxin) producing isolates gave any signal in the PCR assay. The assay turned out to be highly specific for emetic strains, no false positive nor any false negative signal was detected.

Figure 2 shows results of the PCR amplification of target DNA with the above mentioned primer combination of the invention (upper part of the figure) and results of control PCR using standard primer targeting 16 S rDNA (lower part of the figure).

### 2. Example: The detection of emetic Bacillus cereus in microbial mixtures

### Microbial DNA mixtures.

The specificity of the designed primers was determined with a mixture of chromosomal DNA isolated from different bacterial species which are well known as food borne pathogens. One mixture contained all members of the *B*. *cereus* group *(B. cereus* ATCC 14579^{T}, *B. anthracis* Sterne, CIP7702, *B*. *thuringiensis* WS2614, B. *weihenstephanensis* WSBC10204 ^{T}, *B. mycoides* WSBC10276, *B. pseudomycoides* WS3118), a second mixture contained different types of *Staphylococcus aureus* (WS2604 (SEA); WS2606 (SEB); WS2608 (SEC); WS2610 (SED); WS2612 (SEE)) and a third mixture contained *Bacillus licheniformis* (WSBC23001); *Clostridium perfringens* (ATCC3628); *Staphylococcus aureus* (WS2604); *Staphylococcus equorum* (WS2733); *Listeria monocytogenes* (WSLC10209); *E. coli* (WS2577); *Salmonella Dublin* (WS2692); *Yersinia enterocolitica* (WS2589).

### PCR amplification.

PCR mixture (50 µl) contained 0.8 mM dNTPs mix (ABgene, Epsom, U.K), 1.5 mM MgCl₂, 50 pM of each primer, 1.25 U of ThermoStart *Taq* DNA polymerase (ABgene, Epsom, U.K.), 5 µl 10x polymerase buffer (ABgene, Epsom, U.K) and 1 µl DNA mixture (as described above). Thermal cycling was carried out in a Peqlab thermocycler, Cyclone Gradient (Biotechnologie GmbH, Germany).The PCR protocol started with a denaturation step for 15 min at 95°C, followed by 30 cycles of 1 min 95°C, 1.5 min 60°C, 50 s at 72°C each, and ended with a final elongation step at 72°C for 5 min.

### Detection of amplicons.

PCR products were separated on a 1% agarose gel, stained with ethidium bromide and visualized with Image Master VDS (Pharmacia Biotech).

### Evaluation of the PCR assay.

When the target nucleic acid consisted of chromosomal DNA from a mixture of food borne pathogens, the primer 800F2 and 800R2 amplified only a fragment in the present of emetic *Bacillus cereus* DNA (Fig. 3).

### References

Aas N., B. Gondrosen, and G. Langeland 1992. Norwegian food authorities report on food associated diseases in 1990, SNT report 3, Oslo.
Agata, N., M. Mori, M. Ohta, S. Suwan, I. Ohtani, and M. Isobe. 1994. A novel dodecadepsipeptide, cereulide, isolated from *Bacillus cereus* causes vacuole formation in HEp-2 cells. FEMS Microbiol. Lett. 121: 31 - 34.
Agata N., M. Ohta, M. Mori, and M. Isobe. 1995. A novel dodecadepsipeptide, cereulide, is an emetic toxin of *Bacillus cereus.* FEMS Microbiol Lett. **129**:17-20.
Akiyama N., K. Mitani, Y. Tanaka, Y. Hanazono, N. Motoi, M. Zarkovic, T. Tange, H. Hirai, and Y. Yazaki. 1997. Fulminant septicemic syndrome of *Bacillus cereus* in a leukemic patient. Intern Med. **36**: 221 - 226.
Altschul S.F., W. Gish, W. Miller, E.W. Myers, and D.J. Lipman. 1990. Basic local alignment search tool. J Mol Biol **215** : 403 - 410.
Andersson, M., R. Mikkola, J. Helin, M. C. Andersson, and M. Salkinoja-Salonen. 1998. A novel sensitive bioassay for detection of *B. cereus* emetic toxin and related depsipeptide ionophores. Appl. Environ. Microbiol. **64**: 1338 - 1343.
Anonymous 1999. Molecular diagnostic methods for infectious diseases. Approved guideline MM3-A. NCCLS, Wayne, PA
Anonymous. 2001. WHO surveillance program for control of food borne infections and intoxications in Europe (Seventh report 1993 - 1998). Ed. K. Schmidt and C. Tirado. Berlin, Germany. 45.
Beecher, D. J., T. W. Olsen, E. B. Somers, and A. C. L. Wong. 2000. Evidence for contribution of tripartite hemolysin BL, phosphatidylcholine-preferring phospholipase C, and collagenase to virulence of *Bacillus cereus* endophthalmitis. Infect. Immun. 68: 5269-5276.
Drowniewski, F. A. 1993. *Bacillus cereus* and related species. Clin. Microbiol. Rev. 6: 324-338.
Ehling-Schulz M., B. Svensson, M.-H. Guinebretiere, T. Lindbäck, M. Andersson, A. Schulz, A. Christionsson, P. E. Granum, E. Märtlbauer, C. Nyguyen-The, M. S. Salkinoja-Salonen, and S. Scherer. (2003). Mapping of Toxin Producing *Bacillus cereus* Revealed a Single Cluster for Emetic Strains, submitted
Finlay, W. J., N. A. Logan, and A. D. Sutherland. 1999. Semiautomated metabolic staining assay for *Bacillus cereus* emetic toxin. Appl. Environ. Microbiol. 65: 1811 - 1812.
Ginsburg A. S., L. G. Salazar, L. D. True, and M. L. Disis. 2003 Fatal *Bacillus cereus* sepsis following resolving neutropenic enterocolitis during the treatment of acute leukemia. Am J Hematol. 72: 204 - 208.
Girisch M., M. Ries, M. Zenker, R. Carbon, R. Rauch, and M. Hofbeck. 2003. Intestinal perforations in a premature infant caused by *Bacillus cereus.* Infection. **31**: 192 - 193.
Granum, P. E., and T. Lund. 1997. *Bacillus cereus* enterotoxins. FEMS Microbial. Lett. **157**: 223 - 228.
Granum, P. E.. 2001. *Bacillus cereus.* In: Food microbiology: Fundamentals and Frontiers, Ed. Doyle M. P. et al., 373 -381; ASM Press, Washington, D.C.
Guinebretiere, M. H., V. Broussolle, and C. Nguyen-The. 2002. Enterotoxigenic profiles of food-poisoning and food-borne *Bacillus cereus* strains. J. Clin. Microbiol. **40**: 3053 - 3056.
Häggblom M.M., C Apetroaie, MA Andersson, and M.S. Salkinoja-Salonen. 2002. Quantitative analysis of cereulide, the emetic toxin of *Bacillus cereus,* produced under various conditions. Appl Environ Microbiol. **68**: 2479 - 2483.
Hall J. A., J. S. Goulding, N. H. Bean, R. V. Tauxe, and C. W. Hedberg. 2001. Epidemiologic profiling: evaluating foodborne outbreaks for which no pathogen was isolated by routine laboratory testing: United States, 1982-9. Epidemiol Infect. **127**: 381 - 387.
Hansen, B. M., and N. B. Hendriksen. 2001. Detection of enterotoxic *Bacillus cereus* and *Bacillus thuringiensis* strains by PCR analysis. Appl. Environ. Microbiol. **67**: 185 - 189.
Hatakka M.. 1998. Microbiological quality of hot meals served by airlines. J Food Prot. **61**: 1052 - 1056.
Helgason, E., D. A. Caugant, I. Olsen, and A.-B. Kolstø. 2000. Genetic structure of population of *Bacillus cereus* and *Bacillus thuringiensis* associated with periodontitis and other human infections. J. Clin. Microbiol. **38**: 1615 - 1622.
Hilliard N. J., R. L. Schelonka, and K. B. Waites. 2003. *Bacillus cereus* bacteremia in a preterm Neonate. J. Clin. Microbiol. **41**: 3441 - 3444.
Hoornstra D., M. A. Andersson, R. Mikkola, and M.S. Salkinoja-Salonen. 2003. A new method for in vitro detection of microbially produced mitochondrial toxins. Toxicol in Vitro, in press.
Inglis G.D. and Kalischuk L.D. 2003. Use of PCR for direct detection of *Campylobacter* species in bovine feces. Appl. Environ. Microbiol. 69:3435-3447
Innis M. A., D. J. Gelfand, J. J. Sninsky, and T. J. White. 1990. PCR protocols. A guide to methods and applications. Academic Press Inc., Harcourt Brace Jovanovich Publishers, USA.
Isobe M., T. Ishikawa, S. Suwan, N. Agata and M. Ohta. 1995. Synthesis and activity of Cereulide, a cyclic dodecadepsipeptide ionophore as emetic toxin from *Bacillus cereus*. Bioorganic & Medicinal Chemistry Letters. **5**: 2855 - 2858.
Kleinkauf H., and H. Von Döhren. 1996. A nonribosomal system of peptide biosynthesis. Eur J Biochem. **236**: 335 - 351.
Kramer J. M., and R. J. Gilbert. 1989. *Bacillus cereus* and other *Bacillus* species. In: M. P. Doyle (Ed.), Food borne bacterial pathogens, Marcel Dekker Inc., New York, 21 - 70.
Lantz P.-G., Al-Soud W.A., Knutsson R., Hahn-Hägerdal B., Rådström P. 2000. Biotechnical use of polymerase chain reaction for microbiological analysis of biological samples. Biotechnol. Annu. Rev. 5:87-130
Lund, T., M. L. De Buyser, and P. E. Granum. 2000. A new cytotoxin from *Bacillus cereus* that may cause necrotic enteritis. Mol. Microbiol. 38: 254 - 261.
Mahler, H., A. Pasi, J. M. Kramer, P. Schulte, A. C. Scoging, W. Bär, and S. Krähenbühl. 1997. Fulminant liver failure in association with the emetic toxin of *Bacillus cereus.* N. Engl. J. Med. 336: 1142-1148.
Maniatis T., Fritsch E.F., Sambrook J. 1998. Molecular cloning: A laboratory manual. Cold Spring Harbour, N.Y.: Cold Spring Harbor Laboratory
Marahiel, M. A., T. Stachelhaus and H. D. Mootz. 1997. Modular peptide synthetases involved in nonribosomal peptide synthesis. Chem Rev. 97: 2651 - 2673.
Mikkola R., N.E. Saris, P.A.Grigoriev, M.A. Andersson, and M.S. Salkinoja-Salonen. 1999. Ionophoretic properties and mitochondrial effects of cereulide: the emetic toxin *of B. cereus*. Eur J Biochem. **263**: 112 - 117.
McPherson M. J., B. D. Hames, and G. R. Tylor. 1995. PCR, a practical approach, Vol. 2, Information Press Ltd., Oxon, Great Britain.
Musa M.O., M. Al Douri, S. Khan,T. Shafi, A. Al Humaidh, and A. M. Al Rasheed. 1999. Fulminant septicaemic syndrome of *Bacillus cereus:* three case reports. J Infect. **39**: 154 - 156.
Paananen A., R.Mikkola, T. Sareneva, S. Matikainen, M. Hess, M. Andersson, I. Julkunen, M. S. Salkinoja-Salonen, and T. Timonen. 2002. Inhibition of human natural killer cell activity by cereulide, an emetic toxin from *Bacillus cereus.* Clin Exp Immunol. **129**: 420 - 428.
Prüß, B. M., R. Dietrich, B. Nibler, E. Märtelbauer, and S. Scherer. 1999. The hemolytical enterotoxin HBL is broadly distributed among species of the *Bacillus cereus* group. Appl. Environ Bacteriol. 65: 5436-5442.
Ramesh A., Padmapriya, B.P., Chandrashekar A., Varadaraj M.C. 2002. Application of a convenient DNA extraction method and multiplex PCR for direct detection of *Staphylococcus aureus* and *Yersinia enterocolitica* in milk samples. Mol. Cell. Probes. 16:307-314
Shinagawa K., H. Konuma, H. Sekita, and S. Sugii. 1995. Emesis of rhesus monkeys induced by intragastric administration with the HEp-2 vacuolation factor (cereulide) produced by *Bacillus cereus.* FEMS Microbiol Lett. **130**: 87 - 90.
Shinagawa K., Y. Ueno, D. Hu, S. Ueda and S. Sugii. 1996. Mouse lethal activity of a HEp-2 vacuolation factor, cereulide, produced by *Bacillus cereus* isolated from vomiting-type food poisoning. J Vet Med Sci. 58: 1027 - 1029.
Stachelhaus T., and M. A. Marahiel. 1995. Modular structure of genes encoding multifunctional peptide synthetases required for non-ribosomal peptide synthesis. FEMS Microbiol Lett. **125**: 3 - 14.
Stackebrandt, E., and W. Liesack. 1992. The potential of rDNA in identification and diagnostics. In Nonradioactive Labeling and Detection of Biomolecules, pp. 232-239. Ed. by C. Kessler. Berlin, Springer.
Thuler L.C., E. Velasco, C. A. de Souza Martins, L. M. de Faria, N. P. da Fonseca, L. M. Dias, and V. M. Goncalves. 1998. An outbreak of *Bacillus* species in a cancer hospital. Infect Control Hosp Epidemiol. **19**: 856 -858.
Tsai Y.-L. and Olson B.H. 1991. Rapid method for direct extraction of DNA from soil and sediments by polymerase chain reation. Appl. Environ. Microbiol. 58:2292-2295 Von Döhren, H., U. Keller, J. Vater and R. Zocher. 1997. Multifunctional Peptide Synthetases. Chem Rev. **97**: 2675 - 2705.

## Claims

1. Oligonucleotide primers which, in combination, amplify a specific DNA fragment of emetic *Bacillus cereus* strains.

a) Primer 1 (800F2): 5′-GACAAGAGAAATTTCTACGAGCAAGTACAAT-3′
b) Primer 2 (800R2): 5′-GCAGCCTTCCAATTACTCCTTCTGCCACAGT-3′
(seq no. 1 and 2)

2. The oligoprimers according to claim 1, **characterized in that** one of the primers comprises the nucleotide sequence a) of claim 1 and the other primer comprises the nucleotide sequence b) of claim 1.

3. The oligoprimers according to claim 1, **characterized in that** one of the primers comprises 5' end modifications.

4. The nucleic acid sequence of the amplified gene product of emetic *B*. *cereus* that is amplified with the primers of claim 1 (seq no. 3)

5. The use of the nucleic acid sequence of claim 4 to design PCR primers for detection of emetic *B*. *cereus,* including primers for RT PCR and multiplex PCR.

6. The use of the nucleic acid sequence of claim 4 to design probes for detection of emetic *B*. *cereus* using hybridization techniques.

7. A method for detection emetic *B*. *cereus* strains in any sample, **characterized in that** the method comprises the following steps:
1. Collection of sample.
2. Preparation of sample for nucleic acid amplification.
3. Adding reagents for nucleic acid amplification to the prepared sample, including oligonucleotide primers that in combination amplify a chromosomal DNA fragment of emetic *B*. *cereus* strains and at least on of the oligonucleotides comprises one of the nucleotide sequences of claim 1 or a modified derivative of it.
4. Detection of the presence of emetic *B*. *cereus.*

8. A kit to detect emetic *B*. *cereus* in any sample.

9. Use of these sequences in an assay/kit which differentiates between enterotoxigenic *Staphylococcus aureus* and emetic *Bacillus cereus.*

10. Use of the sequence information of claim **4** for the construction of peptide antibodies to detect emetic *B*. *cereus* in any sample.


SEQUENCE LISTING

<110> Scherer, Siegfried

<120> Assay for detection of emetic toxin producing Bacillus cereus

<130> 1

<160> 3

<170> PatentIn version 3.1

<210> 1
<211> 31
<212> DNA
<213> Bacillus cereus

<400> 1
gacaagagaa atttctacga gcaagtacaa t 31

<210> 2
<211> 31
<212> DNA
<213> Bacillus cereus

<400> 2
gcagccttcc aattactcct tctgccacag t 31

<210> 3
<211> 635
<212> DNA
<213> Bacillus cereus

<220>
<221> gene
<222> (1)..(635)

<223>

<400> 3
gacaagagaa atttctacga gcaagtacaa tcatttttct gctcatgata ttgctttaaa 60
ctggatgccg ttagaacatg ttggcggtat agtaatgttc catattaaag atacttattt 120
agggcgaaat caagtacaag tgagaacgca atatgtttta tcagaaccga caagatggct 180
tgatcttatc acaacttaca aagcgacaat tacttgggca ccaaactttg cttttgcttt 240
aattaacaag gaaatcgaga atggtgttaa gggtaattgg gacttatcat caatggagtt 300
tattgtaaat gccggcgaag caattaacgg ttatacagca aagaaatttc tacaagtatt 360
gtcaccgtat ggcttgccag aagatgcaat gattccagta tgggggatgt cagaaacatg 420
ttcaggtgtt gtatataata aagcatttcg atcggatgga accaaaggac tatttattgt 480
agacaaacaa tctttagaag gcattataca agaagggaat aacgtggagg acaccatcac 540
atttgttaac ttaggaaaac ccatatatgg tgtctctatc cgaattgcta atcaagataa 600
tgagactgtg gcagaaggag taattggaag gctgc 635